Europäisches Patentamt

European Patent Office

Office européen des brevets

(19)

(11) Veröffentlichungsnummer: **0 161 337**
**B1**

(12) **EUROPÄISCHE PATENTSCHRIFT**

(45) Veröffentlichungstag der Patentschrift: 30.05.90

(21) Anmeldenummer: 84114959.4

(22) Anmeldetag: 08.12.84

(51) Int. Cl.⁵: **A 61 K 6/08,** C 09 J 133/10, C 08 F 220/00, C 07 F 9/09

(54) **Photopolymerisierbarer phosphathaltiger dentaler Haftvermittler-Lack.**

(30) Priorität: 14.04.84 DE 3414163

(43) Veröffentlichungstag der Anmeldung:
21.11.85 Patentblatt 85/47

(45) Bekanntmachung des Hinweises auf die Patenterteilung:
30.05.90 Patentblatt 90/22

(84) Benannte Vertragsstaaten:
CH DE FR GB LI

(56) Entgegenhaltungen:
EP-A-0 088 527
AT-B- 350 693
DE-A-3 139 235

CHEMICAL ABSTRACTS, Band 98, Nr. 24, 13. Juni 1983, Columbus, Ohio, USA SANKIN INDUSTRY CO., LTD "Dental and bone cements containing acryloyloxyalkyl pyrophosphates" Seite 377, Spalte 1, Zusammenfassung-Nr. 204 455k

(73) Patentinhaber: Kulzer GmbH
Philipp-Reis-Strasse 8
D-6393 Wehrheim (TS.)1 (DE)

(72) Erfinder: Janda, Ralf, Dr.
Seulbergerstrasse 19
6380 Bad Homburg (DE)
Erfinder: Eppinger, Bernhard
Ringstrasse 26
6393 Wehrheim 3 (DE)

(74) Vertreter: Heinen, Gerhard, Dr.
Heraeusstrasse 12-14
D-6450 Hanau/Main (DE)

(56) Entgegenhaltungen:
CHEMICAL ABSTRACTS, Band 98, Nr. 5, 31. Jänner 1983, Columbus, Ohio, USA SANKIN INDUSTRY CO., LTD. "Pyrophosphoric acid esters" Seite 665, Spalte 2, Zusammenfassung-Nr. 34 758s

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft einen dentalen Haftvermittler-Lack aus einer Lösung eines Methacryloyloxyäthylphosphats in einem flüchtigen organischen Lösungsmittel.

Beim Füllen von Zahnkavitäten mit einem polymerisierbaren Zahnfüllungsmaterial auf Kunststoff-Basis wird zur Verbesserung der Haftung zwischen Zahnschmelz und Kunststoff der Zahnschmelz vor dem Legen der Füllung mit einer sauren Ätzlösung, zum Beispiel einer verdünnten Phosphorsäure-Lösung, behandelt und gegebenenfalls dann noch mit einem dünnflüssigen, meist monomere Dimethacrylate enthaltenden chemisch oder durch Photopolymerisation auszuhärtenden Versiegelungsmaterial bestrichen. Wegen ihrer guten physikalischen Eigenschaften haban sich Versiegelungsmaterialien, die zusätzlich zu den Monomeren einen feinteiligen anorganischen Füllstoff enthalten, besonders bewährt. Ihre Haftung beruht auf einer mechanischen Verankerung im geätzten Zahnschmelz.

Ein Ätzen des Dentins empfiehlt sich aus zahnärztlicher Sicht jedoch nicht. Daher werden, wenn eine Verankerung durch Schmelzätzung nicht möglich ist, zum Beispiel bei Zahnhalskavitäten, zur Verbesserung der Haftung zwischen Dentin und Kunststoff Haftmittel verwendet. Eine Vielzahl solcher dentaler Haftmittel ist bekannt.

In Adhesive Restorative Dental Materials 1961, 195—198, wird über die haftverbessernde Wirkung von Bis-(methacryloyloxyäthyl)-hydrogenphosphat und einigen Methacryloyloxy-Gruppen enthaltenden Dihydrogenphosphaten berichtet.

Aus der europäischen Patentanmeldung 88 527 ist ein die Haftung verbesserndes Material ("adhesion promoter"), das aus einer Lösung eines mindestens drei Methacryloyloxy-Gruppen aufweisenden Dihydrogenphosphats in einem flüchtigen Lösungsmittel, besonders einem Alkanol, besteht, bekannt. Zusammen mit diesem Material kann, um die Haftung des Zahnfüllungsmaterials an der Zahnsubstanz zu verstärken, ein Haft- oder Versiegelungsmaterial ("intermediate bonding resin") verwendet werden.

Aus den veröffentlichten japanischen Patentanmeldungen 57/56 490 und 57/167 364 ist die Verwendung von Tetramethacryloyloxyäthylpyrophosphat in Dentalmaterialien bekannt (Chemical Abstracts Vol. 98, 1983, 34 758 und 204 455).

Es ist die Aufgabe der Erfindung, ein Mittel zu finden, das durch chemische Bindung die Haftung eines photopolymerisierbaren Versiegelungsmaterials am Dentin bewirkt.

Diese Aufgabe wird erfindungsgemäß durch einen Haftvermittler-Lack, der aus einer Lösung von Methacryloyloxyäthyl-dihydrogenphosphat und/oder Bis-(methacryloyloxyäthyl)-hydrogenphosphat und eines Photopolymerisationskatalysators in Aceton besteht, gelöst.

Vorzugsweise enthält der Haftvermittler-Lack 10—30 Gewichts-% des Phosphats oder der Phosphate, besonders 15—20 Gewichts-%.

Als Photopolymerisationskatalysatoren haben sich die aus der GB—PS 1 408 265 bekannten Gemische aus Ketonen und Aminen, besonders aus Campherchinon und Aminen oder aus Campherchinon, Benzoylverbindungen, zum Beispiel Benzilacetale oder Benzoylalkanole, und Aminen, zum Beispiel 4-Dimethylaminobenzoesäure-2-butoxyäthylester, bewährt.

Vorzugsweise enthält der Haftvermittler-Lack jeweils 0,1—0,5 Gewichts-% des Campherchinons, des Amins und der Benzoylverbindung.

Durch die Anwendung des Haftvermittler-Lackes gemäß der Erfindung zusammen mit einem photopolymerisierbaren Versiegelungsmaterial läßt sich eine haft- und dauerfeste Verbindung zwischen Dentin und dem Zahnfüllungsmaterial auf Kunststoff-Basis schaffen.

Das in dem in dünner Schicht auf das Dentin gestrichenen Haftvermittler-Lack vorhandene Aceton bewirkt eine gute Benetzung der Dentinoberfläche und eine gleichmäßige Verteilung des Lackes. Ein weiterer Vorteil des Acetons ist es, daß mit seinem Verdunsten auch am Dentin vorhandende Spuren von Feuchtigkeit entfernt werden. Das nach dem Verdunsten des Acetons aufzutragende einen Photopolymerisationskatalysator enthaltende Versiegelungsmaterial wird durch Bestrahlung mit UV-Licht, vorzugsweise jedoch durch Bestrahlung mit sichtbarem Licht ausgehärtet.

Mit dem einen Photopolymerisationskatalysator enthaltenden Haftvermittler-Lack gemäß der Erfindung wird — wahrscheinlich aufgrund einer vollständigen Copolymerisation des Methacryloyloxyäthylphosphats mit den Monomeren des Versiegelungsmaterials — eine höhere Haftfestigkeit als mit einem keinen Katalysator dieser Art enthaltenden Lack erreicht.

Zur näheren Erläuterung wird in dem folgenden Beispiel die Herstellung von Haftvermittler-Lacken gemäß der Erfindung beschrieben.

2

Die mit diese Lacken unter Verwendung eines photopolymerisierbaren Versiegelungsmaterials erreichte Haftfestigkeit zwischen Dentin und Kunststoff-Zahnfüllung wird bestimmt.

Beispiel

Durch Vermischen von

a) 79,5 g Aceton, absolut
   20,0 g Methacryloyloxyäthyl-dihydrogenphosphat
   0,1 g 1,2-Diphenyl-2,2-dimethoxyäthanon
   0,3 g Campherchinon und
   0,1 g 4-Dimethylaminobenzoesäure-2-butoxyäthylester
und von

b) 79,5 g Aceton, absolut
   20,0 g Bis-(methacryloyloxyäthyl)-hydrogenphosphat
   0,1 g 1,2-Diphenyl-2,2-dimethoxyäthanon
   0,3 g Campherchinon und
   0,1 g 4-Dimethylaminobenzoesäure-2-butoxyäthylester
werden die Haftvermittler-Lacke A und B hergestellt.

Jeweils einer dieser Lacke wird auf die mit einer Diamantsäge plan gesägte, polierte und getrocknete Dentinoberfläche von extrahierten Zähnen aufgestrichen. Nach dem Verdunsten des Acetons wird auf die erhaltene Lack-Schicht eine dünne Schicht des photopolymerisierbaren Versiegelungsmaterials Durafill bond der Firma Kulzer aufgetragen, durch 20 Sekunden langes Bestrahlen mit dem Halogenlichtgerät der Firma Kulzer (Translux®) ausgehärtet und mit dem photopolymerisierbaren Zahnfüllungsmaterial Durafill der Firma Kulzer bedeckt, das durch 20 Sekunden langes Bestrahlen mit dem Halogenlichtgerät ausgehärtet wird.

Die so behandelten Zähne werden zur Bestimmung der Haftfestigkeit zwischen Dentin und Kunststoff Abscherversuchen unterworfen. Die dabei unmittelbar nach Aushärtung des Zahnfüllungsmaterials, nach 7tägiger und nach 40tägiger Lagerung in 37°C warmem Wasser erhaltenen Werte werden in der Tabelle angegeben.

TABELLE

| Haftver- mittler- Lack | Haftfestigkeit (N/mm$^2$) | |
|---|---|---|
| | unmittelbar nach Aus- härtung | nach 7 tägiger Lagerung in Wasser, 37°C |
| A | 5,5 | 4,0 |
| B | 6,2 | 5,0 |

**Patentansprüche**

1. Dentaler Haftvermittler-Lack aus einer Lösung eines Methacryloyloxyäthylphosphats in einem flüchtigen organischen Lösungsmittel, dadurch gekennzeichnet, daß er aus einer Lösung von Methacryloyloxyäthyl-dihydrogenphosphat und/oder Bis-(methacryloyloxyäthyl)-hydrogenphosphat und eines Photopolymerisationskatalysators in Aceton besteht.

2. Haftvermittler-Lack nach Anspruch 1, dadurch gekennzeichnet, daß er 10—30 Gewichts-% des Phosphats oder der Phosphate enthält.

3. Haftvermittler-Lack nach Anspruch 2, dadurch gekennzeichnet, daß er 15—20 Gewichts-% des Phosphats oder der Phosphate enthält.

4. Haftvermittler-Lack nach einem der Ansprüche 1—3, dadurch gekennzeichnet, daß der Photopolymerisationskatalysator aus Keton und Amin besteht.

5. Haftvermittler-Lack nach Anspruch 4, dadurch gekennzeichnet, daß der Photopolymerisationskatalysator aus Campherchinon und Amin besteht.

6. Haftvermittler-Lack nach Anspruch 4, dadurch gekennzeichnet, daß der Photopolymerisationskatalysator aus Campherchinon, Benzoylverbindung und Amin besteht.

**Revendications**

1. Vernis adhésif dentaire comprenant une solution d'un phosphate de méthacryloyloxyéthyle dans un solvant organique volatil, caractérisé en ce qu'il consiste en une solution de dihydrogénophosphate de méthacryloyloxyéthyle et/ou d'hydrogénophosphate de bis-(méthacryloyloxyéthyle) et d'un catalyseur de photopolymérisation dans l'acétone.

2. Vernis adhésif selon la revendication 1, caractérisé en ce qu'il contient 10—30% en poids du phosphate ou des phosphates.

3. Vernis adhésif selon la revendication 2, caractérisé en ce qu'il contient 15—20% en poids du phosphate ou des phosphates.

4. Vernis adhésif selon l'une des revendications 1—3, caractérisé en ce que le catalyseur de photopolymérisation consiste en cétone et amine.

5. Vernis adhésif selon la revendication 4, caractérisé en ce que le catalyseur de photopolymérisation consiste en camphoquinone et amine.

6. Vernis adhésif selon la revendication 4, caractérisé en ce que le catalyseur de photopolymérisation consiste en camphoquinone, composé benzoylé et amine.

**Claims**

1. A dental bonding agent lacquer consisting of a solution of a methacryloyloxyethyl phosphate in a volatile organic solvent, characterised in that it consists of a solution of methacryloyloxyethyl-dihydrogen phosphate and/or of bis-(methacryloyloxyethyl)-hydrogen phosphate and a photopolymerisation catalyst in acetone.

2. A bonding agent lacquer according to claim 1, characterised in that it contains 10 to 30% by weight of the phosphate or phosphates.

3. A bonding agent lacquer according to claim 2, characterised in that it contains 15 to 20% by weight of the phosphate or phosphates.

4. A bonding agent lacquer according to one of the claims 1 to 3, characterised in that the photopolymerisation catalyst consists of ketone and amine.

5. A bonding agent lacquer according to claim 4, characterised in that the photopolymerisation catalyst consists of camphor quinone and amine.

6. A bonding agent lacquer according to claim 4, characterised in that the photopolymerisation catalyst consists of camphor quinone, benzoyl compound and amine.